# EUROPÄISCHE PATENTANMELDUNG

(11) **EP 0 753 580 A2**
(43) Veröffentlichungstag der Anmeldung: **15.01.1997**
(21) Anmeldenummer: 96110962.6
(22) Anmeldetag: 08.07.1996
(51) Int. Cl.: C12N 15/85, A61K 48/00

(54) **Zellspezifische Gentherapie mit Hilfe eines neuen Promotors für den "Tissue Inhibitor of Metalloproteinase-3"**

(30) Priorität: 12.07.1995 DE 19524720
(71) Anmelder: HOECHST AKTIENGESELLSCHAFT, 65929 Frankfurt am Main (DE)
(72) Erfinder: Sedlacek, Hans-Harald, Prof. Dr., 35041 Marburg (DE); Wick, Marisa, 35041 Marburg (DE); Müller, Rolf, Prof. Dr., 35037 Marburg (DE)

(57) **Zusammenfassung**

Die Erfindung betrifft Promotorsequenzen für das Gen des 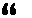 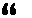 Tissue Inhibitors of Metalloproteinase-3" (TIMP-3). Diese Inhibitor kommt besonders in Makrophagen und Synovialzellen der Gelenke vor.

## Beschreibung

Die Erfindung betrifft Promotorsequenzen für das Gen des "Tissue Inhibitors of Metalloproteinase-3" (TIMP-3). Dieser Inhibitor kommt besonders in Makrophagen und Synovialzellen der Gelenke vor.

Einer der wesentlichen Probleme der in vivo Verabreichung von Vektoren für die Gentherapie ist die zielzellspezifische Expression des verabreichten Vektors. Grundsätzlich wird diese beispielsweise erreicht durch ein zellspezifisches Promotorelement, welches durch zellspezifische Transkriptionsfaktoren aktiviert wird und eine am 3' Ende sich anschließende Gensequenz aktiviert (Übersichten bei Mullen, Pharmac. Ther. 63, 199 (1994), Harris et al., Gene Therapy 1, 170 (1994)). Es besteht somit ein großes Bedürfnis nach neuen zellspezifischen Promotoren.

Die Erfindung wird wie folgt näher beschrieben:

### 1. Isolierung und Sequenzanalyse der 5'-flankierenden Promotorsequenz des humanen TIMP-3-Gens

Die Induktion der TIMP-3-mRNA-Expression während der G0→S-Progression beruht hauptsächlich auf einer Aktivierung der Transkription des TIMP-3-Gens (Wick et al., J. Biol. Chem. 269, 18963 (1994)). Die 5'-flankierende Sequenz des menschlichen TIMP-3-Gens wurde kloniert, der Startpunkt der Transkription der TIMP-3-mRNA bestimmt und der angrenzende Promotorbereich einer Struktur-Funktions-Analyse unterzogen. Diese Untersuchungen sollten die Regulationsmechanismen klären, die der spezifischen TIMP-3-Expression während der G0→ S- und G1→ S-Progression zugrundeliegen.

Durch eine genomische Southern Blot-Analyse wurde vorab bestimmt, ob TIMP-3 im menschlichen Genom ein Einzelgen darstellt oder mehrere Loci für das TIMP-3-Gen bzw. eventuell auch TIMP-3-Pseudogene existieren. Hierzu wurde genomische DNA aus WI-38-Zellen isoliert, mit den Restriktionsendonukleasen EcoRI, PstI und HindIII behandelt und einer Southern Blot-Analyse unterzogen. Als radioaktiv-markierte Sonde wurde ein 690 bp langes 3'-TIMP-3-cDNA-Fragment eingesetzt. Da die Sonde in allen Fällen nur ein spezifisches DNA-Fragment erkannte, ist davon auszugehen, daß nur ein singuläres TIMP-3-Gen im menschlichen Genom vorliegt.

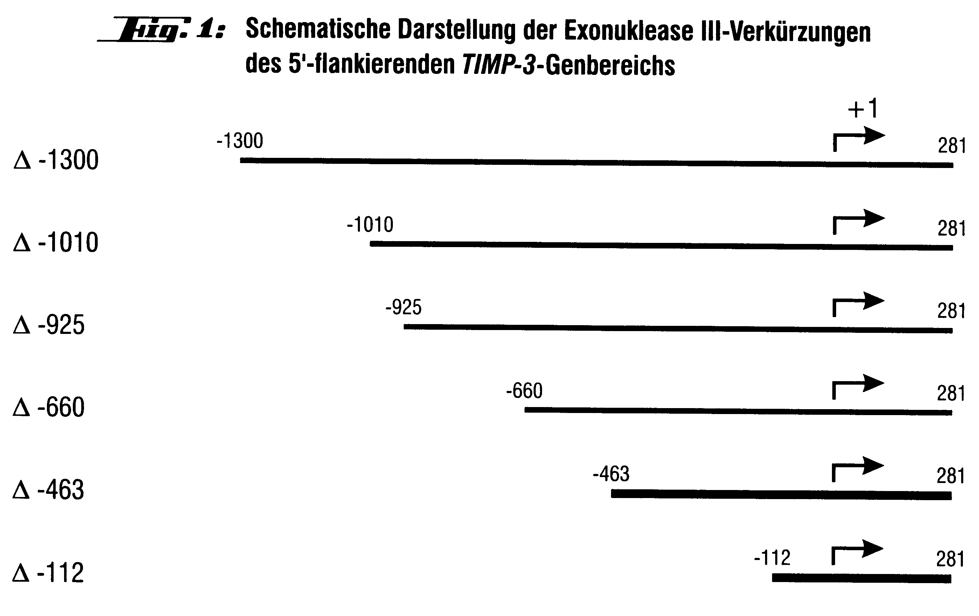

Zur Isolierung der 5'-flankierenden TIMP-3-Gensequenz wurden ca. 7x10⁵ Phagen einer genomischen WI-38-Genbibliothek mit einem 300 bp langen 5'-TIMP-3-cDNA-Fragment hybridisiert. Von den dreizehn nach dieser Primäruntersuchung isolierten rekombinanten Phagenklonen wurden vier auch von einem 30 bp langen Oligonukleotid aus dem 5'-Endbereich der TIMP-3-cDNA erkannt. Da diese Phagen wahrscheinlich auch den das ATG-Startcodon flankierenden, 5'-Sequenzbereich enthielten, wurde einer der Phagenklone für eine nähere Charakterisierung und Analyse ausgewählt. Durch kombinierte Behandlung mit verschiedenen Restriktionsendonukleasen und nachfolgender Southern Blot-Analyse konnte bestimmt werden, daß das 13 kb lange genomische DNA-"Insert" dieses Phagen ca. 4,7 kb der 5'-flankierenden TIMP-3-Gensequenz enthielt. Durch Sequenzanalyse beider Stränge wurde die Nukleotidsequenz von annähernd 1500 bp des 5'-flankierenden Genbereichs bestimmt. Die hierfür durch eine Exonuklease III-Behandlung hergestellten 5'-Verkürzungen des klonierten 5'-Genbereichs sind in Abb. 1 illustriert.

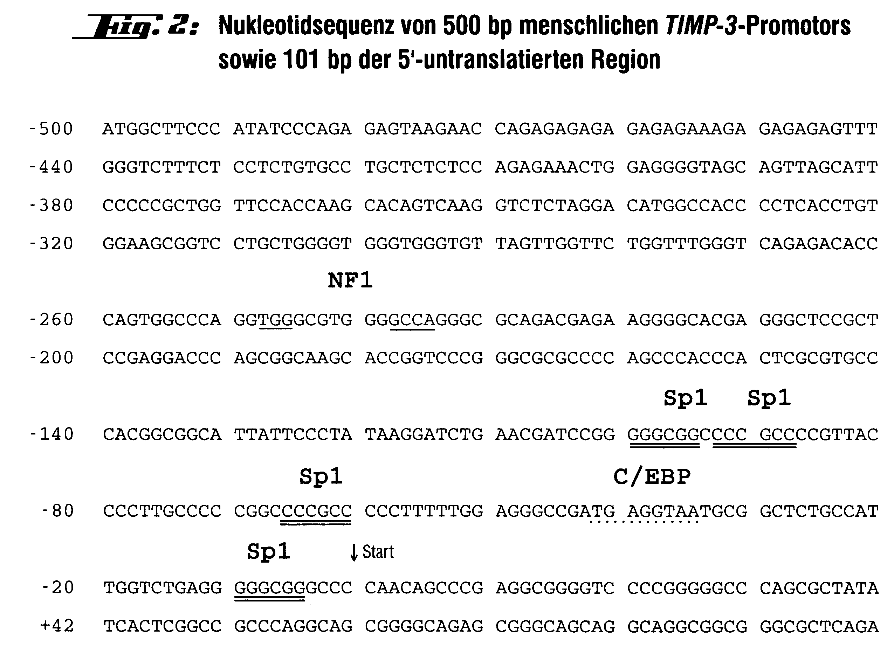

Der Sequenzbereich, der sich durch die nachfolgend beschriebenen Struktur-Funktions-Analysen als besonders bedeutend für die TIMP-3-Promotor-Funktion herausstellte, ist in Abb. 2 gezeigt. Durch Computer-gestützte Analyse wurden in der TIMP-3-Promotorsequenz eine Reihe von Elementen identifiziert, die den Bindungsstellen bekannter Transkriptionsfaktoren ähneln, u.a. 4 Sp1-Bindungsstellen, eine mögliche NF1- sowie eine C/EBP-Bindungsstelle (markiert in Abb. 2).

### 2. Kartierung des Transkriptionsstartpunktes der TIMP-3-mRNA

Um den oder die Startpunkt(e) der Transkriptionsinitiation zu ermitteln, wurde das 5'-Ende der TIMP-3-mRNA durch eine Primer-Extension-Analyse bestimmt. Hierbei wurde eine Transkriptionsstartstelle (Nucleotidsequenz: CCGCCCGGGGTTGTCGG) identifiziert, die 364 bp 5' vom ATG-Startcodon lokalisiert ist (markiert in Abb. 2). Trotz genauer Untersuchung der aufwärts der Startstelle gelegenen Nukleotidsequenz wurde weder eine TATA-Box noch TATA-ähnliche Sequenzen gefunden.

### 3. Untersuchungen zur Aktivität der TIMP-3-Promotorsequenz

Um die Aktivität der TIMP-3-Promotorsequenz in normal proliferierenden, ruhenden und serumstimulierte Zellen zu bestimmen und erste Hinweise auf funktionell wichtige Promotorbereiche zu enthalten, wurden die zur Sequenzierung verwendeten 5'-verkürzten Promotorfragmente (s. Abb. 1) vor das Luziferasegen in den promotorlosen pXP-2-Vektor (Nordeen, Biotechniques 6, 454 (1988)) kloniert. Durch seine äußerst geringe Basalaktivität eigent sich dieses "Reporterkonstrukt" besonders zur Durchführung transienter Expressionsanalysen.

### 3.1. Aktivität der TIMP-3-Promotorsequenz in normal proliferierenden und serumstimulierten NIH3T3-Zellen

Um zu zeigen, daß die isolierte TIMP-3-Promotorsequenz in transienten Expressionsanalysen aktiv ist, d.h. die Transkription des LuziferaseReportergens steuern kann, wurde das TIMP-3-Promotor-Deletionskonstrukt - 1010 (umfaßt die Nukleotide - 1010 bis + 281, s. Abb. 1) in NIH3T3-Zellen transfiziert und die Luziferase-Aktivität in diesen normal proliferierenden oder serumstimulierten transfizierten Zellen bestimmt. Zum Vergleich wurde zusätzlich die Expression von weiteren Luziferase-Promotorkonstrukten ermittelt, die den Herpes Virus tk-Promotor (pT81; Lucibello und Müller, Meth. Mol. Cell Biol. 1, 9 (1989)), einen 5xTRE-Minimalpromotor (Angel et al., Mol. Cell Biol. 7, 2256 (1987)), einen RSV-LTR (Setoyama et al., Proc. Natl. Acad. Sci. USA 83, 3213 (1986) oder ein 937 bp langes Fragment des menschlichen Cyclin D1-Promotors (Herber et al., Oncogene 9, 1295 (1994)) enthielten. Die Ergebnisse dieser Untersuchungen sind in Tabelle 1 aufgeführt.

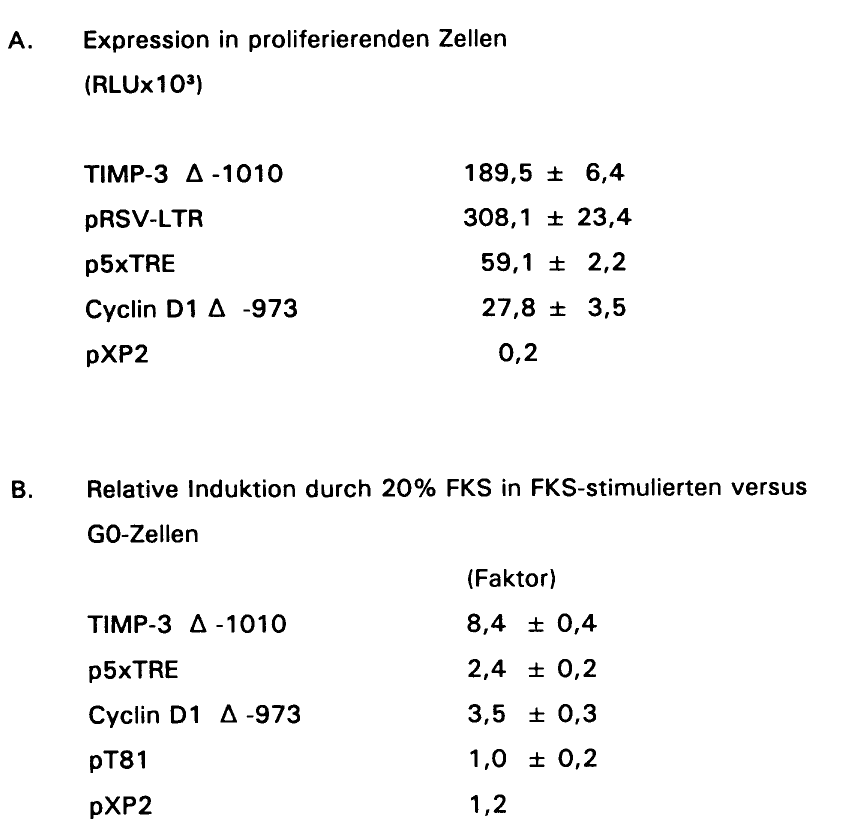

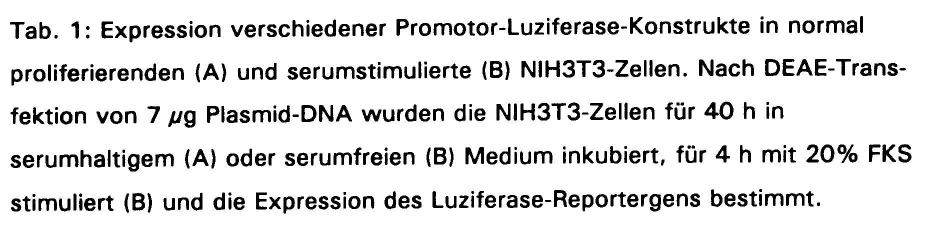

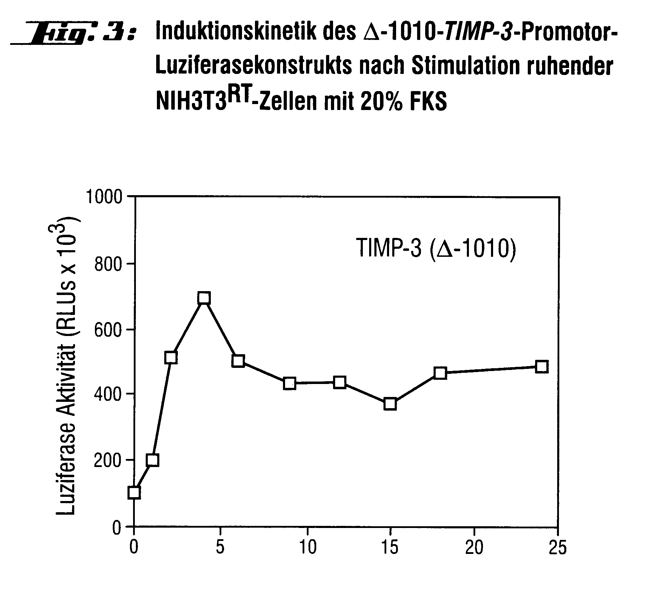

In normal proliferierenden NIH3T3-Zellen (Tab. 1 A) wurde das TIMP-3-Promotorkonstrukt Δ-1010 ca. 3-fach höher exprimiert als der 5xTRE-Minimalpromotor und zeigt eine 7-fach höhere Expression als das Cyclin D1-Promotorkonstrukt. Einzig das RSV-LTR-Reporterplasmid wies eine ca. 2-fach höhere Aktivität als das TIMP-3-Promotorkonstrukt auf. Diese Ergebnisse deuten darauf hin, daß der menschliche TIMP-3-Promotor durch eine vergleichsweise hohe transkriptionelle Aktivität gekennzeichnet ist. Wie in Tab. 1B und Abb. 3 gezeigt, wurde das TIMP-3-Promotorkonstrukt Δ-1010 außerdem deutlich in Zellen induziert, die nach zweitägigem Serumentzug für 4 h mit 20% FKS stimuliert worden waren. Verglichen mit ruhenden (G0) Zellen stieg die Expression dabei ca. 7- bis 8-fach an, was etwa 3,5-fach bzw. 2,4-fach höher lag als die beobachteten Induktionswerte des 5xTRE-Reporterkonstruktes bzw. des Cyclin D1-Promotorkonstrukts. Dagegen zeigte das Herpes simplex tk-Promotor-Luziferasekonstrukt (pT81) keine Induktion seiner Expression nach Serumstimulation.

In Abb. 3 ist die Kinetik der Induktion des Δ-1010 TIMP-3-Promotorkonstrukts nach Serumstimulation ruhender Zellen gezeigt. Die Luziferase-Aktivität stieg bereits nach 1 h an und erreichte mit einer 7-fachen Induktion nach 4 h Maximalwerte.

Zusammenfassend kann man aus diesen Ergebnissen folgern, daß das verwendete Δ-1010 TIMP-3-Promotorkonstrukt die wesentlichen, wenn nicht alle regulatorischen Elemente aufweist, die für eine effiziente Transkription sowie für die Induzierbarkeit durch Serum benötigt werden.

### 3.2 Struktur- und Funktionsanalyse der TIMP-3-Promotorsequenz

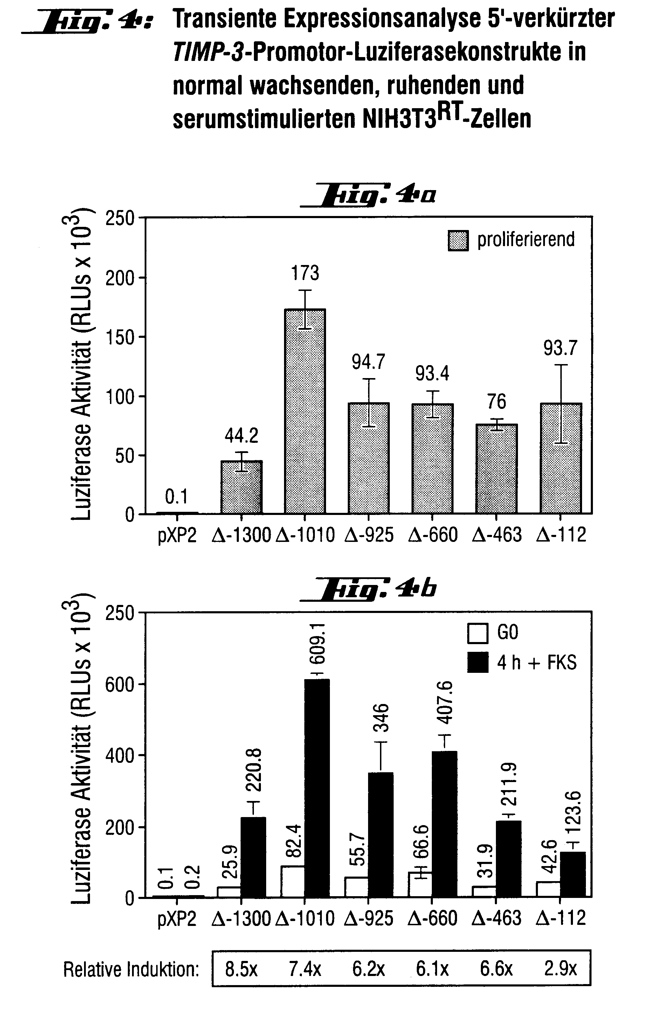

Eine Struktur- und Funktionsanalyse der isolierten TIMP-3-Promotorsequenz sollte erste Hinweise auf diejenigen Promotorregionen liefern, die für die Basalexpression sowie die Seruminduzierbarkeit funktionell bedeutend sind. Hierzu wurde die Aktivität der verschiedenen, für die Promotorsequenzierung hergestellten und in den pXP-2-Vektor umklonierten, TIMP-3-Promotor-Deletionskonstrukte (s. Abb. 1) in transienten Expressionsanalysen bestimmt. Die Analyse der basalen Expression der verschiedenen Deletionskonstrukte in normal proliferierenden NIH3T3-Zellen (Abb. 4) ergab drei wesentliche Ergebnisse:
1. Die stärkste Expression wies das Promotorkonstrukt Δ-1010 auf. Eine Verkürzung um weitere 85 bp (Konstrukt Δ-925) führte zu einem annähernd 2-fachen Absinken der Promotoraktivität. Dies deutet auf die Präsenz von einem oder mehreren Elementen in der Region zwischen Position -1010 und -925 hin, die an der Transkriptionsaktivierung beteiligt sind.
2. Durch weitere Verkürzungen des 5'-Endes bis hin zu Position -112 wurde die Promotoraktivität nicht signifikant beeinflußt. Die Region zwischen - 925 und -112 enthält daher wahrscheinlich keine für die Promotoraktivität wichtigen Sequenzbereiche.
3. Die Region zwischen Position -1300 und -1010 scheint dagegen einen negativen Effekt auf die Promotoraktivität auszuüben, was sich in der im Vergleich zum Promotorkonstrukt Δ-1010 ca. 4-fach reduzierten Expression des Δ-1300 Deletionskonstrukts ausdrückt.

Im abschließenden Experiment wurde die Seruminduzierbarkeit der verschiedenen TIMP-3-Promotor-Deletionskonstrukte analysiert. Die Ergebnisse dieser, wie in Tabelle 1 beschriebenen durchgeführten, Expressionsanalysen sind in Abb. 4b dargestellt. Auffällig ist das ähnliche Expressionsprofil zwischen normal proliferierenden (Abb. 4a), ruhenden und serumstimulierten Zellen (Abb. 4b). Die Expressionswerte in ruhenden Zellen lagen aber ca. 2-fach niedriger als in proliferierenden Zellen und wurden 4 h nach Serumstimulation 2,9- bis 8,5-fach induziert. Wie in den proliferierenden Zellen gezeigt (Abb. 4a), übt die Region zwischen Position Δ -1300 und Δ-1010 einen negativen Effekt auf die Promotoraktivität in ruhenden und serumstimulierten Zellen aus, hat aber keinen Einfluß auf die Seruminduzierbarkeit des Konstruktes Δ-1300 (8,5-fache Induktion). Auch hier wurden die höchsten Luziferaseaktivitäten wieder mit dem Deletionskonstrukt -1010 gemessen. Weitere Verkürzungen des 5'-Endes bis zu Position -660 führten nur zu einem 1,5- bis 2-fachen Absinken der Promotoraktivität. Alle diese Konstrukte (Δ-1300, Δ-1010, Δ-925, Δ-660) zeigten aber eine deutliche, 6- bis 8-fache Induktion nach Serumzugabe. Die Verkürzung um weitere 200 bp bis zu Position -463 (Δ-463) bewirkte ein erneutes 2-faches Absinken der Aktivität, hatte aber ebenfalls keinen Einfluß auf die Seruminduzierbarkeit des Konstrukts. Erst das Konstrukt Δ-112 zeigte mit einem nur 3-fachen Expressionsanstieg nach Serumstimulation eine 50-65%ige Reduktion seiner Seruminduzierbarkeit. Dies deutet darauf hin, daß die Region zwischen Position -463 und -112 Element(e) enhält, die für die Seruminduzierbarkeit des TIMP-3-Promotors von Bedeutung sind. Zusätzliche Regionen zwischen Position -463 und -660 sowie -925 und -1010 verstärken generell und zellzyklusunabhängig die seruminduzierte Promotoraktivität.

Die Ergebnisse der Charakterisierung sowie Struktur- und Funktionsanalyse des 5'-flankierenden TIMP-3-Genbereiches lassen sich wie folgt zusammenfassen:

TIMP-3 stellt ein TATA-Box-loses Gen dar. Die Transkription wird aber dennoch an nur einer Startstelle 364 bp aufwärt vom ATG-Startcodon initiiert. Verglichen mit anderen Promotoren weist die TIMP-3-Promotorsequenz eine relativ hohe Aktivität auf, für die die ersten 112 bp ausreichend sind. In dieser Region befinden sich zahlreiche Sp1-Bindungsstellen. Außerdem zeigt er eine deutliche Induktion seiner Aktivtät nach Serumstimulation ruhender Zellen, deren Kinetik der TIMP-3-mRNA-Expression während der G0→ S-Progression entspricht. Die für die Seruminduzierbarkeit verantwortlichen Regulationselemente sind in der Region zwischen Position -112 und -463 lokalisiert.

Legende zu Fig. 1 - 4:
Fig. 1:
   Schematische Darstellung der Exonuklease III-Verkürzungen des 5'-flankierenden TIMP-3-Genbereichs.
   Zur Erleichterung der Sequenzierung wurden annähernd 1600 bp des 5'-flankierenden TIMP-3-Genbereichs durch Behandlung mit Exonuklease III vom 5'-Ende her verkürzt und in den Bluescript SK (-)-Vektor kloniert. Die Namen der Plasmide bezeichnen ihre 5'-Verkürzung (z.B. Δ-1300 enthält 1300 bp 5' der Transkriptionsstartstelle). Die Transkriptionsstartstelle ist mit + 1 markiert (vergl. Fig. 2).
Fig. 2:
   Nukleotidsequenz von 500 bp menschlichen TIMP-3-Promotors sowie 101 bp der 5'-untranslatierten Region.
   GC-Boxen (Sp1-Bindungsstellen), zwei Halbseiten einer möglichen NF1-Bindungsstelle sowie ein der C/EBP-Bindungstelle ähnelndes Element sind markiert. Die Transkriptionsstartstelle ist durch einen Pfeil gekennzeichnet.
Fig. 3:
   Induktionskinetik des Δ-1010-TIMP-3-Promotor-Luziferasekonstrukts nach Stimulation ruhender NIH3T3^{RT}-Zellen mit 20 % FKS.
   Graphische Darstellung. Nach DEAE-Transfektion von 7 µg Plasmid-DNA wurden die NIH3T3^{RT}-Zellen für 40 h in serumfreiem Medium inkubiert, mit 20 % FKS stimuliert und zu den angegebenen Zeitpunkten die Expression des Luziferase-Reportergens bestimmt.
Fig. 4:
   Transiente Expressionsanalyse 5'-verkürzter TIMP-3-Promotor-Luziferasekonstrukte in normal wachsenden, ruhenden und serumstimulierten NIH3T3^{RT}-Zellen.

Die Plasmide wurden entsprechend ihrer Verkürzungen bezeichnet (s. Fig. 1)
(a) Analyse in normal wachsenden NIH3T3-Zellen.
(b) Analyse der gleichen Konstrukte wie in (a) in ruhenden versus für 4 h mit 20 % FKS stimulierten NIH3T3-Zellen.

Die Experimente in (a) und (b) wurden, wie in Tab. 1 beschrieben, dreimal mit unabhängig voneinander präparierten Plasmid-DNAs durchgeführt. Standardabweichungen sind als Balken dargestellt. Ein Fehlen der Balken indiziert eine sehr niedrige im Graph nicht mehr darstellbare Standardabweichung.

## Patentansprüche

1. Promotor für das Gen des Tissue Inhibitor of Metalloproteinase-3, enthaltend promotoraktive DNA-Fragmente enthaltend Position ≦ 463 bis ≧ -2 der folgenden Nukleotidsequenz:

-500 ATGGCTTCCC ATATCCCAGA GAGTAAGAAC CAGAGAGAGA GAGAGAAAGA GAGAGAGTTT
-440 GGGTCTTTCT CCTCTGTGCC TGCTCTCTCC AGAGAAACTG GAGGGGTAGC AGTTAGCATT
-380 CCCCCGCTGG TTCCACCAAG CACAGTCAAG GTCTCTAGGA CATGGCCACC CCTCACCTGT
-320 GGAAGCGGTC CTGCTGGGGT GGGTGGGTGT TAGTTGGTTC TGGTTTGGGT CAGAGACACC

NF1

-260 CAGTGGCCCA GGTGGGCGTG GGGCCAGGGC GCAGACGAGA AGGGGCACGA GGGCTCCGCT

-200 CCGAGGACCC AGCGGCAAGC ACCGGTCCCG GGCGCGCCCC AGCCCACCCA CTCGCGTGCC

Sp1 Sp1

-140 CACGGCGGCA TTATTCCCTA TAAGGATCTG AACGATCCGG GGGCGGCCCC GCCCCGTTAC

Sp1 C/EBP

-80 CCCTTGCCCC CGGCCCCGCC CCCTTTTTGG AGGGCCGATG AGGTAATGCG GCTCTGCCAT

Sp1 Start

-20 TGGTCTGAGG GGGCGGGCCC CAACAGCCCG AGGCGGGGTC CCCGGGGGCC CAGCGCTATA

2. Promotor für das Gen des Tissue Inhibitor of Metalloproteinase-3, enthaltend
Position ≦ 463 bis ≧ -10
oder
Position ≦ 112 bis ≧ - 2
oder
Position ≦ 112 bis ≧ -10
der unter Anspruch 1) aufgeführten Nukleotidsequenz.

3. Verwendung des Promotors nach Anspruch 1) oder 2) für die Regulation der Expression eines Gens.

4. Verwendung des Promotors nach Anspruch 1-3) für die Herstellung eines Arzneimittels.

5. Verwendung des Promotors nach Anspruch 1-3) für ein diagnostisches Verfahren.

6. Verwendung des Promotors nach Anspruch 1-3) für die Gentherapie.


SEQUENZPROTOKOLL

(1) ALLGEMEINE INFORMATION:

(i) ANMELDER:
(A) NAME: Hoechst Aktiengesellschaft
(B) STRASSE: -
(C) ORT: Frankfurt
(D) BUNDESLAND: -
(E) LAND: Germany
(F) POSTLEITZAHL: 65926
(G) TELEPHON: 069-305-3005
(H) TELEFAX: 069-35-7175
(I) TELEX: 4 1 234 700 ho d

(ii) ANMELDETITEL: Zellspezifische Gentherapie mit Hilfe eines
neuen Promotors fuer den "Tissue Inhibitor of
Metalloproteinase-3"

(iii) ANZAHL DER SEQUENZEN: 1

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 540 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..540

(xi) SEQUENZBESCHREIBUNG: SEQ ID NO: 1:

-500 ATGGCTTCCC ATATCCCAGA GAGTAAGAAC CAGAGAGAGA GAGAGAAAGA GAGAGAGTTT

-440 GGGTCTTTCT CCTCTGTGCC TGCTCTCTCC AGAGAAACTG GAGGGGTAGC AGTTAGCATT

-380 CCCCCGCTGG TTCCACCAAG CACAGTCAAG GTCTCTAGGA CATGGCCACC CCTCACCTGT

-320 GGAAGCGGTC CTGCTGGGGT GGGTGGGTGT TAGTTGGTTC TGGTTTGGGT CAGAGACACC

-260 CAGTGGCCCA GGTGGGCGTG GGGCCAGGGC GCAGACGAGA AGGGGCACGA GGGCTCCGCT

-200 CCGAGGACCC AGCGGCAAGC ACCGGTCCCG GGCGCGCCCC AGCCCACCCA CTCGCGTGCC

-140 CACGGCGGCA TTATTCCCTA TAAGGATCTG AACGATCCGG GGGCGGCCCC GCCCCGTTAC

- 80 CCCTTGCCCC CGGCCCCGCC CCCTTTTTGG AGGGCCGATG AGGTAATGCG GCTCTGCCAT

- 20 TGGTCTGAGG GGGCGGGCCC CAACAGCCCG AGGCGGGGTC CCCGGGGGCC CAGCGCTATA